(19) Europäisches Patentamt
European Patent Office
Office européen des brevets

(11) **EP 3 695 411 B1**

(12) **EUROPEAN PATENT SPECIFICATION**

(45) Date of publication and mention
of the grant of the patent:
**10.07.2024 Bulletin 2024/28**

(21) Application number: **18799895.0**

(22) Date of filing: **01.10.2018**

(51) International Patent Classification (IPC):
*A61B 5/021* (2006.01)    *A61B 5/024* (2006.01)
*A61B 5/00* (2006.01)    *G16H 50/30* (2018.01)
*G16H 40/63* (2018.01)

(52) Cooperative Patent Classification (CPC):
**G16H 40/63; A61B 5/02108; A61B 5/7239;**
**A61B 5/7278; A61B 5/746; G16H 50/30;**
A61B 5/024

(86) International application number:
**PCT/US2018/053746**

(87) International publication number:
**WO 2019/074706 (18.04.2019 Gazette 2019/16)**

(54) **CARDIAC MONITORING SYSTEM**

KARDIALES ÜBERWACHUNGSSYSTEM

SYSTÈME DE SURVEILLANCE CARDIAQUE

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**

(30) Priority: **12.10.2017 US 201762571645 P**
        **26.09.2018 US 201816142527**

(43) Date of publication of application:
**19.08.2020 Bulletin 2020/34**

(73) Proprietor: **Edwards Lifesciences Corporation**
**Irvine, CA 92614 (US)**

(72) Inventors:
• **JIAN, Zhongping**
**Irvine**
**CA 92614 (US)**
• **AL HATIB, Feras**
**Irvine**
**CA 92614 (US)**

(74) Representative: **Eisenführ Speiser**
**Patentanwälte Rechtsanwälte PartGmbB**
**Postfach 31 02 60**
**80102 München (DE)**

(56) References cited:
**EP-A2- 2 416 701**     **US-A1- 2014 163 401**

**Description**

BACKGROUND

Cardiac monitoring can be an important part of patient care in the hospital setting, particularly for patients who are seriously ill or undergoing surgery. Many cardiac parameters of significant clinical importance can be derived from central arterial blood pressure waveforms obtained through continuous or periodic blood pressure monitoring. Examples of such significant cardiac parameters include cardiac output, stroke volume, and stroke volume variation, to name a few. Accurately calculating those cardiac parameters can be of critical importance for clinicians responsible for making treatment decisions.

For patients in a normal hemodynamic condition, the relationship between arterial blood pressure measured peripherally and central arterial blood pressure is understood, so that blood pressure measurements taken at the femoral or radial arteries, for example, can be used to quickly and accurately determine cardiac output and stroke volume, as well as other cardiac parameters of interest. However, for patients in a hyperdynamic condition, peripheral arterial blood pressure and central arterial blood pressure may be decoupled, rendering conventional solutions for determining cardiac parameters based on peripheral arterial blood pressure measurements unreliable.

US 2014/0163401 A1 discloses a method for determining a beat-to-beat stroke volume and/or a cardiac output based on a measurement of suitable arterial pressure data. A waveform of the arterial pressure pulse is assessed based on data obtained during the measurement. A compliance or impedance in dependence of at least one measurement of arterial pressure data is computed using a non-linear model that comprises an arctangent model. The arctangent model is differentiated numerically or analytically to obtain the compliance or the impedance of an aortic portion. The thus obtained compliance or impedance is then substituted into a Windkessel model or a Waterhannner model. As a result, the beat-to-beat stroke volume and/or cardiac output are computed.

EP 2 416 701 A2 describes a method for monitoring central-to-peripheral arterial pressure decoupling, i.e., hyperdynamic conditions. The method involves the comparison of parameters calculated from multivariate statistical models established for both subjects experiencing normal hemodynamic conditions and subjects experiencing hyperdynamic conditions, in which central-to-peripheral decoupling may occur. The difference or ratio between the parameters calculated using the two multivariate statistical models is described as providing a continual indication of the level of decoupling as well as indicating peripheral decoupling when a threshold value is exceeded.

SUMMARY

There are provided systems and methods for performing cardiac monitoring, substantially as shown in and/or described in connection with at least one of the figures, and as set forth more completely in the claims.

BRIEF DESCRIPTION OF THE DRAWINGS

Figure 1 shows a diagram of an exemplary cardiac monitoring system, according to one implementation;

Figure 2A shows an exemplary diagram comparing central arterial blood pressure waveforms to peripheral arterial blood pressure waveforms for a patient in a normal hemodynamic condition;

Figure 2B shows an exemplary diagram comparing central arterial blood pressure waveforms to peripheral arterial blood pressure waveforms for a patient in a hyperdynamic condition;

Figure 3A shows an exemplary diagram comparing a distribution of systolic areas of arterial blood pressure waveforms measured in patients in a normal hemodynamic condition to a distribution of systolic areas of arterial blood pressure waveforms measured in patients in a hyperdynamic condition;

Figure 3B shows an exemplary diagram comparing a distribution of systolic time for arterial blood pressure waveforms measured in patients in a normal hemodynamic condition to a distribution of systolic time for arterial blood pressure waveforms measured in patients in a hyperdynamic condition;

Figure 4A shows an exemplary implementation for non-invasively detecting peripheral arterial blood pressure at an extremity of a patient;

Figure 4B shows an exemplary implementation for performing minimally invasive detection of peripheral arterial blood pressure of a patient;

Figure 5 shows a diagram depicting transformation of a peripheral arterial blood pressure waveform of a patient to a central arterial blood pressure waveform of the patient, according to one implementation;

Figure 6 is a flowchart presenting an exemplary method for use by a system to perform cardiac monitoring; and

Figure 7 shows a trace of an arterial blood pressure waveform including exemplary cardiovascular metrics.

DETAILED DESCRIPTION

The following description contains specific information pertaining to implementations in the present disclosure. One skilled in the art will recognize that the present disclosure may be implemented in a manner different from that specifically discussed herein. The drawings in the present disclosure and their accompanying detailed description are directed to merely exemplary implementations. Unless noted otherwise, like or corresponding elements among the figures may be indicated by like or corresponding reference numerals. Moreover, the drawings and illustrations in the present disclosure are generally not to scale, and are not intended to correspond to actual relative dimensions.

As stated above, cardiac monitoring can be an important part of patient care in the hospital setting, particularly for patients who are seriously ill or undergoing surgery. Many cardiac parameters of significant clinical importance can be derived from central arterial blood pressure waveforms obtained through continuous or periodic blood pressure monitoring. Examples of such significant cardiac parameters include cardiac output, stroke volume, and stroke volume variation, to name a few. Accurately calculating those cardiac parameters can be of critical importance for clinicians responsible for making treatment decisions.

As further stated above, for patients in a normal hemodynamic condition, the relationship between arterial blood pressure measured peripherally and central arterial blood pressure is understood, so that blood pressure measurements taken at the femoral or radial arteries, for example, can be used to quickly and accurately determine cardiac output and stroke volume, as well as other cardiac parameters of interest. However, for patients in a hyperdynamic condition, peripheral arterial blood pressure and central arterial blood pressure may be decoupled, rendering conventional solutions for determining cardiac parameters based on peripheral arterial blood pressure measurements unreliable.

The present disclosure provides systems and methods for performing cardiac monitoring that overcomes the deficiencies of conventional approaches when applied to patients in a hyperdynamic condition. By identifying multiple cardiovascular metrics of a patient based on blood pressure data for the patient, and using a first subset of those cardiovascular metrics supplemented by an additive factor, the present solution enables reliable cardiac monitoring for patients in a clinical setting. Moreover, by determining the additive factor based on a meta-parameter including a weighted sum of combinatorial parameters that include a second subset of the cardiovascular metrics, the present solution advantageously further enables the reliable determination of cardiac output and/or stroke volume for patients whether in a normal hemodynamic or hyperdynamic condition.

It is noted that, as defined for the purposes of the present disclosure, the expression "normal hemodynamic condition" refers to a patient condition in which peripheral arterial blood pressure corresponds to central arterial blood pressure in a way that is known or predictable in the conventional art. As a result, for patients in a normal hemodynamic condition, peripheral arterial blood pressure measurements may be used to reliably calculate central arterial blood pressure, as well as cardiac parameters such as stroke volume and cardiac output that are determined based on central arterial blood pressure.

By contrast, and as further defined for the purposes of the present disclosure, the expression "hyperdynamic condition" refers to a patient condition in which the normal correspondence between peripheral arterial blood pressure and central arterial blood pressure cannot be relied upon. In other words, in a hyperdynamic condition, central arterial blood pressure and peripheral arterial blood pressure are decoupled. Consequently, for patients in a hyperdynamic condition, peripheral arterial blood pressure measurements cannot be reliably used to calculate central arterial blood pressure, nor to determine cardiac parameters such as stroke volume and cardiac output using conventional techniques.

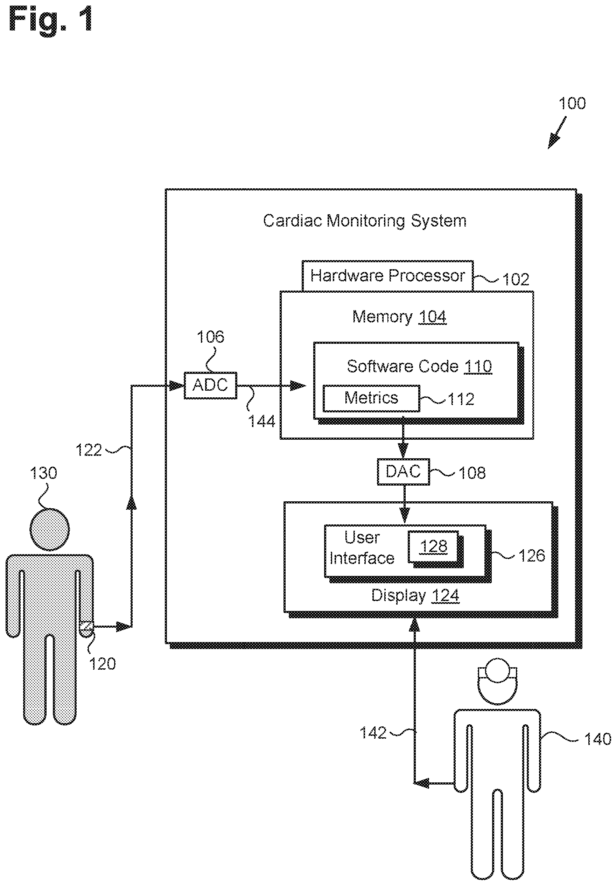

Figure 1 shows a diagram of exemplary cardiac monitoring system 100, according to one implementation. Cardiac monitoring system 100 includes hardware processor 102, system memory 104, and blood pressure sensor 120 coupled to analog-to-digital converter (ADC) 106. As shown in Figure 1, system memory 104 stores software code 110 including cardiovascular metrics 112. As further shown in Figure 1, cardiac monitoring system 100 also includes display 124 providing user interface 126, digital-to-analog converter (DAC) 108, and sensory alarm 128. Display 124 may take the form of a liquid crystal display (LCD), a light-emitting diode (LED) display, an organic light-emitting diode (OLED) display, or another suitable display screen that performs a physical transformation of signals to light.

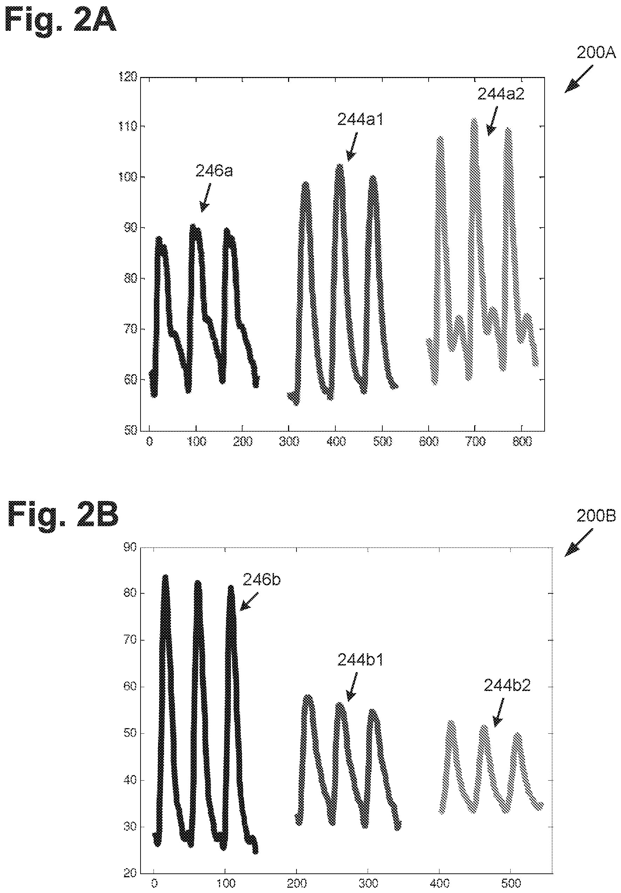

Referring to Figure 2A, Figure 2A shows diagram 200A comparing central arterial blood pressure waveform 246a to peripheral femoral arterial blood pressure waveform 244a1 and peripheral radial arterial blood pressure 244a2 for a patient when in a normal hemodynamic condition. Figure 2B, shows diagram 200B comparing central arterial blood pressure waveform 246b to peripheral femoral arterial blood pressure waveform 244b1 and peripheral radial arterial blood pressure 244b2 for a patient when in a hyperdynamic condition.

As shown in Figure 2A, in a normal hemodynamic condition, the systolic peaks of peripheral femoral arterial blood pressure waveform 244a1 and peripheral radial arterial blood pressure 244a2 are substantially higher than the systolic peaks of central arterial blood pressure waveform 246a. By contrast, in a hyperdynamic condition, those relationships may be inverted, such that the systolic peaks of peripheral femoral arterial blood pressure waveform 244a1 and peripheral radial arterial blood pressure 244a2 are substantially lower than the systolic peaks of central arterial blood pressure waveform 246a. Thus, a hyperdynamic condition may result in a sudden and/or significant reduction in peripheral arterial blood pressure of a patient.

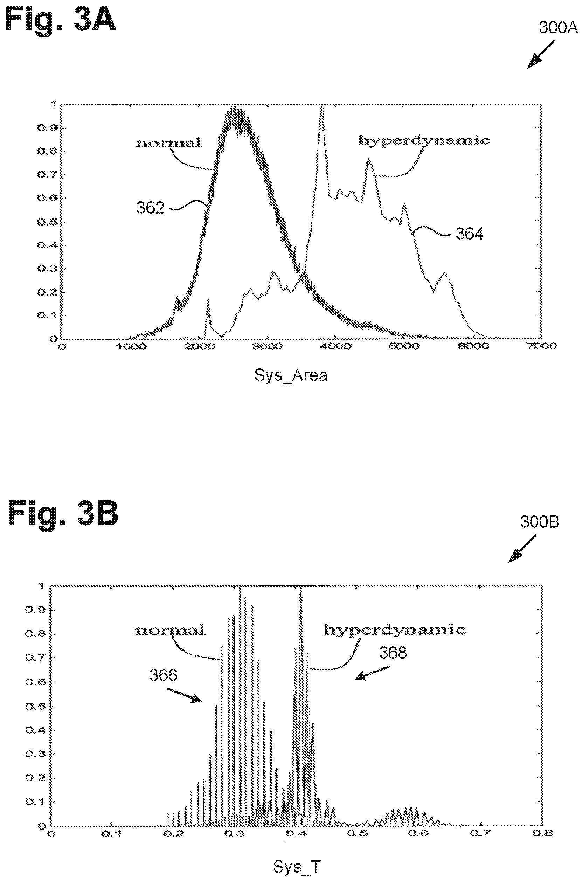

Referring to Figure 3A, Figure 3A shows diagram 300A comparing distribution 362 of systolic areas of arterial blood pressure waveforms measured in patients in a normal hemodynamic condition to distribution 364 of systolic areas of arterial blood pressure waveforms measured in patients in a hyperdynamic condition. As may be seen from Figure 3A, a hyperdynamic condition is associated with a significant shift in systolic area, as well as with a significant change in the standard deviation of distribution 364 relative to distribution 362. Thus, a hyperdynamic condition may result in a sudden and/or significant shift in average systolic area, and/or by a sudden and/or significant increase in the standard deviation of the systolic area distribution.

Figure 3B shows diagram 300B comparing distribution 366 of systolic time of arterial blood pressure waveforms measured in patients in a normal hemodynamic condition to distribution 368 of systolic time of arterial blood pressure waveforms measured in patients in a hyperdynamic condition. As may be seen from Figure 3B, a hyperdynamic condition is associated with a significant shift in systolic time, as well as with a significant change in the standard deviation of distribution 368 relative to distribution 366. Thus, a hyperdynamic condition may result in a sudden and/or significant shift in average systolic time, and/or by a sudden and/or significant decrease in the standard deviation of the systolic time distribution.

Turning back to Figure 1, cardiac monitoring system 100 may be implemented within a patient care environment such as an intensive care unit (ICU) or operating room (OR), for example. As shown in Figure 1, in addition to cardiac monitoring system 100, the patient care environment includes patient 130, and healthcare worker 140 (hereinafter "user 140") trained to utilize cardiac monitoring system 100 via user interface 126. User interface 126 is configured to receive inputs 142 from user 140, and can output cardiac status data and/or provide sensory alarm 128.

Blood pressure sensor 120 is shown in an exemplary implementation in Figure 1, and is attached to patient 130. It is noted that blood pressure sensor 120 may be an invasive or non-invasive sensor attached to patient 130. In one implementation, as represented in Figure 1, blood pressure sensor 120 may be attached non-invasively so as to sense a peripheral arterial blood pressure of patient 130, such as arterial blood pressure measured at a wrist or finger of patient 130. Although not explicitly shown in Figure 1, in other implementations, blood pressure sensor 120 may be attached non-invasively to measure a peripheral arterial blood pressure at an ankle or toe of patient 130.

Alternatively, in some implementations, blood pressure sensor 120 may be attached invasively or non-invasively to measure a peripheral arterial blood pressure at a wrist of patient 130 (i.e., radial blood pressure), or a peripheral arterial blood pressure at a thigh of patient 130 (i.e., femoral blood pressure). Blood pressure signal 122 received by ADC 106 of cardiac monitoring system 100 from blood pressure sensor 120 may include signals corresponding to the arterial blood pressure of patient 130, and may include an arterial blood pressure waveform of patient 130.

According to the exemplary implementation shown in Figure 1, system processor 102 is configured to utilize ADC 106 to convert blood pressure signal 122 to blood pressure data 144 in digital form, and to identify multiple cardiovascular metrics 112 based on digital blood pressure data 144. System processor 102 is further configured to determine the stroke volume (hereinafter "SV") of patient 130 using a subset of cardiovascular metrics 112 and an additive factor. As described in greater detail below, the additive factor is based on a meta-parameter including a weighted sum of combinatorial parameters, each combinatorial parameter including another subset of cardiovascular metrics 112.

Moreover, system processor 102 may be further configured to execute software code 110 to invoke sensory alarm 128 if and it is determined that patient 130 is experiencing a cardiovascular crisis or that such a crisis is imminent. In various implementations, sensory alarm 128 may include one or more of a visual alarm, an audible alarm, and a haptic alarm. For example, when implemented to provide a visual alarm, sensory alarm 128 may be invoked as flashing and/or colored graphics shown by user interface 126 on display 124. When implemented to provide an audible alarm, sensory alarm 128 may be invoked as any suitable warning sound, such as a siren or repeated tone. Moreover, when implemented to provide a haptic alarm, sensory alarm 128 may cause one or more components of system 100 to vibrate or otherwise deliver a physical impulse perceptible to user 140.

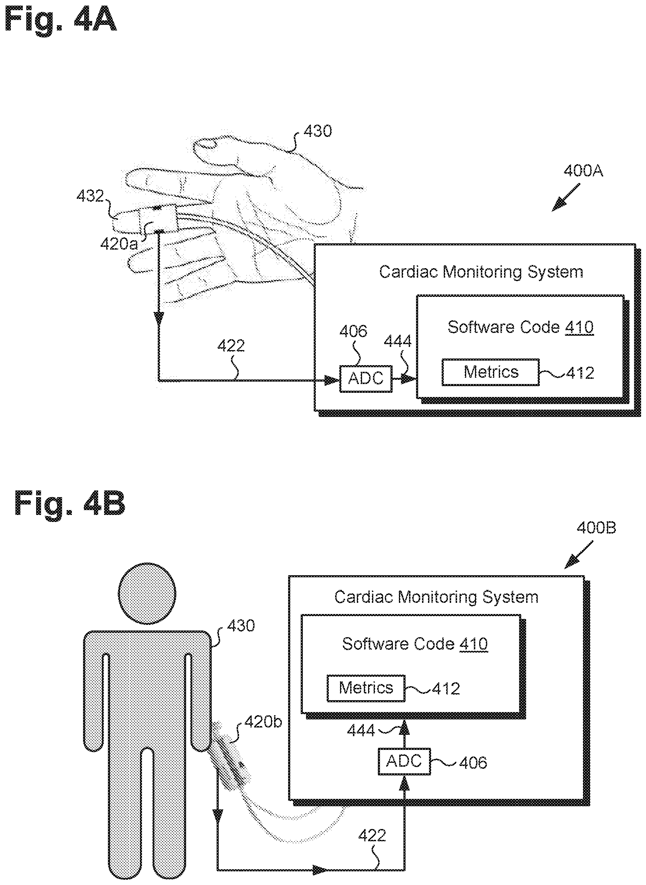

Figure 4A shows an exemplary implementation for sensing peripheral arterial blood pressure non-invasively at an extremity of a patient. Cardiac monitoring system 400A, in Figure 4A, includes ADC 406 and software code 410. As shown by Figure 4A, the arterial blood pressure of patient 430 is sensed non-invasively at finger 432 of patient 430 using blood pressure sensing cuff 420a. Also shown in Figure 4A are blood pressure signal 422 received by ADC 406 of cardiac monitoring system 400A from blood pressure sensing cuff 420a, digital blood pressure data 444 converted from blood pressure signal 422 by ADC 406, and cardiovascular metrics 412 identified based on digital blood pressure data 444 by software code 410.

Patient 430, blood pressure signal 422, and digital blood pressure data 444 correspond respectively in general to patient 130, blood pressure signal 122, and digital blood pressure data 144, in Figure 1, and those corresponding features may share any of the characteristics attributed to either corresponding feature by the present disclosure. Moreover, cardiac monitoring system 400A including blood pressure sensing cuff 420a, ADC 406, and software code 410 including cardiovascular metrics 412, in Figure 4A, corresponds in general to cardiac monitoring system 100 including blood pressure sensor 120, ADC 106, and software code 110 including cardiovascular metrics 112, in Figure 1, and those corresponding features may share any of the characteristics attributed to either corresponding feature by the present disclosure. In other words, although not explicitly shown in Figure 4A, cardiac monitoring system 400A includes features corresponding respectively to hardware processor 102, DAC 108, display 124, user interface 126, and sensory alarm 128.

According to the implementation shown in Figure 4A, blood pressure sensing cuff 420a is designed to sense a peripheral arterial blood pressure of patient 130/430 non-invasively at finger 432 of patient 130/430. Moreover, as shown in Figure 4A, blood pressure sensing cuff 420a may take the form of a small, lightweight, and comfortable blood pressure sensor suitable for extended wear by patient 130/430. It is noted that although blood pressure sensing cuff 420a is shown as a finger cuff, in Figure 4A, in other implementations, blood pressure sensing cuff 420a may be suitably adapted as a wrist, ankle, or toe cuff for attachment to patient 130/430.

It is further noted that the advantageous extended wear capability described above for blood pressure sensing cuff 420a when implemented as a finger cuff may also be attributed to wrist, ankle, and toe cuff implementations. As a result, blood pressure sensing cuff 420a may be configured to provide substantially continuous beat-to-beat monitoring of the peripheral arterial blood pressure of patient 130/430 over an extended period of time, such as minutes or hours, for example.

Figure 4B shows an exemplary implementation for performing minimally invasive detection of peripheral arterial blood pressure of a patient. As shown by Figure 4B, the radial arterial blood pressure of patient 130/430 is detected via minimally invasive blood pressure sensor 420b. It is noted that the features shown in Figure 4B and identified by reference numbers identical to those shown in Figure 4A correspond respectively to those previously described features, and may share any of the characteristics attributed to them above. It is further noted that blood pressure sensor 420b corresponds in general to blood pressure sensor 120, in Figure 1, and those corresponding features may share any of the characteristics attributed to either corresponding feature by the present disclosure.

According to the implementation shown in Figure 4B, blood pressure sensor 420b is designed to sense a peripheral arterial blood pressure of patient 130/430 in a minimally invasive manner. For example, blood pressure sensor 420b may be attached to patient 130/430 via a radial arterial catheter inserted into an arm of patient 130/430. Alternatively, and although not explicitly represented in Figure 4B, in another implementation, blood pressure sensor 420b may be attached to patient 130/430 via a femoral arterial catheter inserted into a leg of patient 130/430. Like non-invasive blood pressure sensing cuff 420a, in Figure 4A, minimally invasive blood pressure sensor 420b, in Figure 4B, may be configured to provide substantially continuous beat-to-beat monitoring of the peripheral arterial blood pressure of patient 130/430 over an extended period of time, such as minutes or hours.

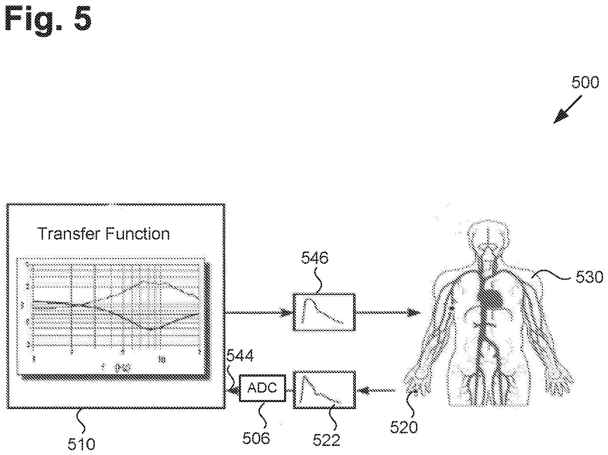

Figure 5 shows diagram 500 depicting transformation of digital blood pressure data 544 converted by ADC 506 from blood pressure signal 522 to central arterial blood pressure waveform 546, according to one implementation. Also shown in Figure 5 are patient 530, blood pressure sensor 520, and software code 310. Blood pressure sensor 320, blood pressure signal 322, ADC 306, digital blood pressure data 544, and software code 510 correspond respectively in general to blood pressure sensor 120/420a/420b, blood pressure signal 122/422, ADC 106/406, digital blood pressure data 144/444, and software code 110/410, in Figures 1, 4A, and 4B, and those corresponding features may share the characteristics attributed to any corresponding feature by the present disclosure.

Thus, blood pressure signal 522 and digital blood pressure data 544 correspond to a peripheral arterial blood pressure waveform of patient 130/430/530 detected using blood pressure sensor 120/420a/420b/520. As shown in Figure 5, digital blood pressure data 144/444/544, converted from blood pressure signal 122/422/522 by ADC 106/406/506, may be transformed to central arterial blood pressure waveform 546 corresponding to an aortic blood pressure waveform or a brachial blood pressure waveform of patient 130/430/530. As further shown by Figure 5, such a transformation may be performed by software code 110/410/510 through application of a transfer function to digital blood pressure data 144/444/544. That is to say, application of such a transfer function may be performed by software code 110/410/510, executed by hardware processor 102.

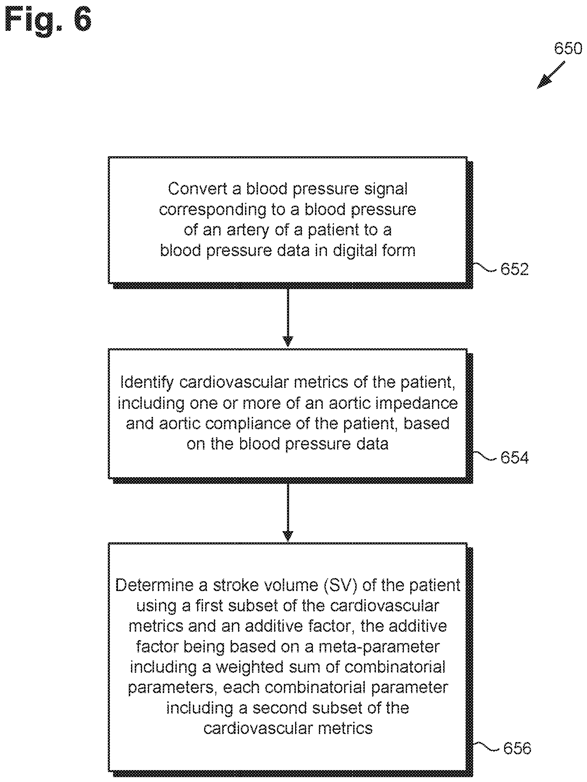

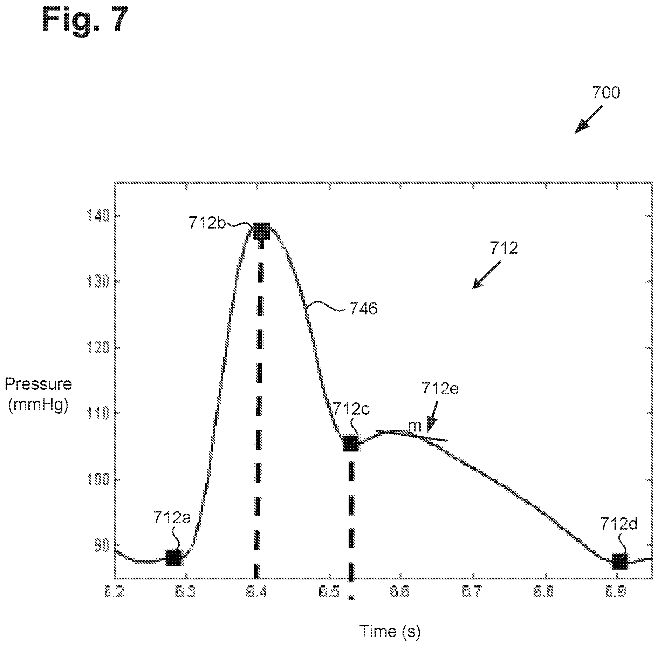

Example implementations of the present disclosure will be further described below with reference to Figures 6 and 7. Figure 6 presents flowchart 650 outlining an exemplary method for use by a system to perform cardiac monitoring. Figure 7 shows a trace of a central arterial blood pressure waveform including exemplary cardiovascular metrics.

Referring to Figure 6 in combination with Figures 1, 4A, 4B, and 5, flowchart 650 begins with converting blood pressure signal 122/422/522 corresponding to a blood pressure of an artery of patient 130/430/530 to blood pressure data 144/444/544 in digital form (action 652). As discussed above, digital blood pressure data 144/444/544 may be converted from blood pressure signal 122/422/522 sensed using blood pressure sensor 120/420a/420b/520 by ADC 106/406/506 of cardiac monitoring system 100/400A/400B. In one implementation, for example, blood pressure sensor 120/420a/420b/520 may be used to sense a peripheral arterial blood pressure of patient 130/430/530, and to generate blood pressure signal 122/422/522 as an analog signal corresponding to that peripheral arterial blood pressure. In such an implementation, ADC 106/406/506 can be used to convert blood pressure signal 122/422/522 into blood pressure data 144/444/544 in digital form.

Flowchart 650 continues with identifying cardiovascular metrics 112/412 of patient 130/430/530 based on digital blood pressure data 144/444/544 of patient 130/430/530 (action 654). As noted above by reference to Figure 5, digital blood pressure data 144/444/544 may be transformed to central arterial blood pressure waveform 546 of patient 130/430/530 by software code 110/410/510, executed by hardware processor 102.

Referring to trace 700 of central arterial blood pressure waveform 746, in Figure 7, it is noted that central arterial blood pressure waveform 746 corresponds in general to central arterial blood pressure waveform 546, in Figure 5, and those corresponding features may share any of the characteristics attributed to either corresponding feature by the present disclosure. As shown in Figure 7, central arterial blood pressure waveform 546/746 may be used to identify various cardiovascular metrics important for performing cardiac monitoring.

For example, trace 700 shows cardiovascular metrics 712 including exemplary cardiovascular metrics 712a, 712b, 712c, and 712d, corresponding respectively to the start of a heartbeat, the maximum systolic blood pressure marking the end of systolic rise, the presence of the dicrotic notch marking the end of systolic decay, and the diastole of the heartbeat of patient 130/430/530. Also included among cardiovascular metrics 712 is exemplary slope 712e of central arterial blood pressure waveform 546/746. It is noted that slope 712e is merely representative of multiple slopes that may be measured at multiple locations along central arterial blood pressure waveform 546/746. It is further noted that cardiovascular metrics 712 correspond in general to cardiovascular metrics 112/412, in Figures 1, 4A, and 4B. Consequently, those corresponding features may share the characteristics attributed to any corresponding feature by the present disclosure.

In addition to cardiovascular metrics 712a, 712b, 712c, and 712d, the behavior of central arterial blood pressure waveform 546/746 during the intervals between those events may also be used as cardiovascular metrics for use in cardiac monitoring. For example, the interval between the start of the heartbeat at cardiovascular metric 712a and the maximum systolic pressure at cardiovascular metric 712b marks the duration of the systolic rise (hereinafter "systolic rise 712a-712b"). The systolic decay of central arterial blood pressure waveform 546/746 is marked by the interval between the maximum systolic pressure at cardiovascular metric 712b and the dicrotic notch at cardiovascular metric 712c (hereinafter "systolic decay 712b-712c"). Together, systolic rise 712a-712b and systolic decay 712b-712c mark the entire systolic phase (hereinafter "systolic phase 712a-712c"), while the interval between the dicrotic notch at cardiovascular metric 712c and the diastole at cardiovascular metric 712d mark the diastolic phase of central arterial blood pressure waveform 546/746 (hereinafter "diastolic phase 712c-712d").

Also of potential clinical interest is the behavior of central arterial blood pressure waveform 546/746 in the interval from the maximum systolic pressure at cardiovascular metric 712b to the diastole at cardiovascular metric 712d (hereinafter "interval 712b-712d"), as well as the behavior of central arterial blood pressure waveform 546/746 from the start of the heartbeat at cardiovascular metric 712a to the diastole at cardiovascular metric 712d (hereinafter "heartbeat interval 712a-712d"). The behavior of central arterial blood pressure waveform 546/746 during intervals: 1) systolic rise 712a-712b, 2) systolic decay 712b-712c, 3) systolic phase 712a-712c, 4) diastolic phase 712c-712d, 5) interval 712b-712d, and 6) heartbeat interval 712a-712d may be identified by measuring the area under the curve of central arterial blood pressure waveform 546/746 and the standard deviation of central arterial blood pressure waveform 546/746 in each of those intervals, for example. The respective areas and standard deviations measured for intervals 1, 2, 3, 4, 5, and 6 above may serve as additional cardiac parameters for use in cardiac monitoring.

It is noted that the standard deviation of central arterial waveform 546/746 across heartbeat interval 712a-712, i.e., interval 6 above, will hereinafter be represented by the lower case Greek letter sigma as "$\sigma$." It is further noted that the area under the curve of central arterial blood and referred to as the pulsatile systolic area in the art will hereinafter be referred to as "PSA."

In addition to the exemplary cardiovascular metrics described above by reference to trace 700, cardiovascular metrics 312/412/712 may include many other metrics. Some examples of those other cardiovascular metrics include the input impedance of the aorta (hereinafter "aortic impedance" or "Z"), as well as a compliance factor corresponding to the compliance of the aorta (hereinafter "aortic compliance" or "C"). It is noted that aortic impedance "Z" and aortic compliance "C" can be determined using various techniques known in the art. For example, a technique for determining Z and/or C using a non-linear model and based on at least one measurement of blood pressure is disclosed in U.S. Patent Application Serial Number 13/896,873 titled "Method, a System and a Computer Program Product for Determining a Beat-to-Beat Stroke Volume and/or a Cardiac Output", filed on May 17, 2013 and published as U.S. Patent Application Publication Number 2014/0163401 A1 on June 12, 2014.

Additional examples of cardiovascular metrics include the pulse rate (hereinafter "PR"), mean arterial blood pressure (hereinafter "MAP"), skewness of the arterial blood pressure (hereinafter "SKU"), and kurtosis of the arterial blood pressure (hereinafter "KURT"). Yet other additional examples of cardiovascular metrics include the whole systolic area (hereinafter "SYS_AREA"), the systolic time (hereinafter "SYS T"), and the diastolic time (hereinafter "DIA T") of arterial blood pressure waveform 546/746 of patient 130/430/530. Identification of cardiovascular metrics 112/412/712 may be performed by software code 110/410/510, executed by hardware processor 102.

Flowchart 650 can conclude with determining the SV of patient 130/430/530 when in the hyperdynamic condition using a first subset of cardiovascular metrics 112/412/712 and an additive factor that is based on a meta-parameter including a weighted sum of combinatorial parameters, each combinatorial parameter including a second subset of cardiovascular metrics 112/412/712 (action 656). For example, the SV of patient 130/430/530 can be determined using the cardiovascular metrics PSA and Z discussed above as follows:

$$\textbf{SV = PSA/Z + } \boldsymbol{\Psi} \qquad \text{(Equation 1)}$$

Where the additive factor is represented by $\Psi$. Thus, in one implementation, the first subset of cardiovascular metrics 112/412/712 includes PSA and Z (i.e., pulsatile systolic area and impedance of the aorta, respectively).

The additive factor $\Psi$ may be expressed as a function of a meta-parameter represented by $\Phi$, wherein:

$$\boldsymbol{\Psi} = (\boldsymbol{\sigma} \textbf{ * } \boldsymbol{\Phi})\textbf{/(MAP-I)} \qquad \text{(Equation 2)}$$

Where $\sigma$ is the standard deviation of central arterial blood pressure waveform 546/746, the symbol * indicates multiplication, MAP is the mean arterial blood pressure of patient 130/430/530, and I is an integer less than 10, such as 5, for example. Thus, in one implementation, additive factor $\Psi$ includes a numerator including meta-parameter $\Phi$, and a denominator including the MAP of patient 130/430/530. Moreover, as shown by Equation 2, in some implementations the numerator of additive factor $\Psi$ is a product of meta-parameter $\Phi$ and $\sigma$.

Thus, substituting Equation 2 into Equation 1, in one implementation:

$$\textbf{SV = (PSA/Z) + ((} \boldsymbol{\sigma} \textbf{ * } \boldsymbol{\Phi})\textbf{/(MAP-I))} \qquad \text{(Equation 3)}$$

As stated above, the meta-parameter $\Phi$ includes a weighted sum of combinatorial parameters, each combinatorial parameter including a second subset of cardiovascular metrics 112/412/712. In one exemplary implementation, the second subset of cardiovascular metrics 112/412/712 may include the following enumerated subset of 10 metrics:

1. $\sigma/10$
2. 600/PR
3. MAP/100
4. SKU
5. KURT + 3
6. (100 * C)/BSA
7. BSA
8. SYS AREA/1000
9. SYS_T * 10
10. DIA T * 10

It is noted that the cardiovascular metrics included in the combinatorial parameters of meta-parameter $\Phi$ may be directly proportional to or inversely proportional to one or more of the cardiovascular metrics identified above prior to the introduction of Equation 1. For example, metric 7 above is directly proportional to the BSA of patient 130/430/530, while metric 6 is inversely proportional to the BSA of patient 130/430/530.

The combinatorial parameters included in $\Phi$ may be obtained from the enumerated subset of cardiovascular metrics 112/412/712 listed above though use of the operator $O$ on that subset. The operator $O$ may be expressed in tabular form as:

$$O = \begin{matrix} m_{11} & m_{12} & m_{13} & Exp_{11} & Exp_{12} & Exp_{13} \\ m_{21} & m_{22} & m_{23} & Exp_{21} & Exp_{22} & Exp_{23} \\ m_{31} & m_{32} & m_{33} & Exp_{31} & Exp_{32} & Exp_{33} \\ m_{41} & m_{42} & m_{43} & Exp_{41} & Exp_{42} & Exp_{43} \end{matrix} \qquad \text{(Equation 4)}$$

Where each row represents a combinatorial parameter. The first three columns of $O$ are an index to the cardiovascular metrics enumerated above, and the last three columns are the exponential power of each cardiovascular metric as it appears in the combinatorial parameter, with the fourth column being the power of the cardiovascular metrics listed in the first column, the fifth column being the power of the cardiovascular metrics listed in the second column, and the sixth column being the power of the cardiovascular metrics listed in the third column.

As a specific example, in one implementation, $O$ may be expressed as:

$$O = \begin{matrix} 3 & 7 & 9 & 1 & 2 & 2 \\ 6 & 7 & 8 & 1 & 2 & 2 \\ 1 & 7 & 9 & -1 & -2 & -1 \\ 2 & 7 & 8 & -1 & 2 & 2 \end{matrix} \qquad \text{(Equation 5)}$$

Equation 5 identifies 4 combinatorial parameters, $P_1$, $P_2$, $P_3$, and $P_4$, corresponding respectively to the first through fourth rows of $O$. Referring to Equation 5 in combination with the enumerated list of cardiovascular metrics above, $P_1$, $P_2$, $P_3$, and $P_4$ may be expressed as:

$$P_1 = (MAP/100) * BSA^2 * (SYS\_T * 10)^2 \qquad \text{(Equation 5-1)}$$

$$P_2 = (100 * C)/BSA * BSA^2 * (SYS\_AREA/1000)^2 \qquad \text{(Equation 5-2)}$$

$$P_3 = (\sigma/10)^{-1} * BSA^{-2} * (SYS\_T * 10)^{-1} \qquad \text{(Equation 5-3)}$$

$$P_4 = (600/PR)^{-1} * BSA^2 * (SYS\_AREA/1000)^2 \qquad \text{(Equation 5-4)}$$

That is to say, the cardiovascular metrics included in one or more of the combinatorial parameters may be raised to an exponential power greater than 1, to a negative exponential power, or to an exponential power more negative than -1 (e.g., - 2).

Meta-parameter $\Phi$ may be expressed in terms of $P_1$, $P_2$, $P_3$, and $P_4$ as:

$$\Phi = C_0 + \sum_{n=1}^{4} (C_n * P_n) \qquad \text{(Equation 6)}$$

Where $C_0$ and the $C_n$ are coefficients that may be determined experimentally. Determination of the SV of patient 130/430/530 using the first subset of cardiovascular metrics 112/412/712 and additive factor $\Psi$ based on meta-parameter $\Phi$ may be performed by software code 110/410/510, executed by hardware processor 102. Moreover, in some implementations, hardware processor 102 is configured to execute software code 110/410/510 to determine the SV of patient 130/430/530 based on the first subset of cardiovascular metrics 112/412/712, additive factor $\Psi$, and using a pulse contour method, as known in the art and disclosed in U.S. Patent Application Serial Number 13/896,873.

In some implementations, the method outlined by flowchart 650 can include invoking, by software code 110/410/510 executed by hardware processor 102, sensory alarm 128 when and if and it is determined that patient 130/430/530 is experiencing a cardiovascular crisis or that such a crisis is imminent. As shown in Figure 3, for example, software code 110/410/510 may be configured to output a cardiac status data to display 324. As further shown in Figure 1, in some implementations, the output of software code 110/410/510 may be processed using DAC 108 to convert digital signals into analog signals for presentation via display 124 and user interface 126.

In addition, in some implementations, the present method may include determining, by software code 110/410/510 executed by hardware processor 102, the cardiac output (hereinafter "CO") of patient 130/430/530 based on the SV. For example, and as known in the art, the CO of a patient in a normal hemodynamic condition may be determined based on the SV of the patient as:

$$CO = SV * PR \qquad \text{(Equation 7)}$$

Where PR is the pulse rate of the patient.

Consequently, the CO of patient 130/430/530 may be obtained through substitution of Equation 3 into Equation

7 as:

$$CO = [PSA/Z + ((\sigma * \Phi)/(MAP\text{-}I))] * PR \qquad \text{(Equation 8)}$$

Thus, the present disclosure provides systems and methods for performing cardiac monitoring that overcome the deficiencies of conventional approaches when applied to patients in a hyperdynamic condition. By identifying multiple cardiovascular metrics including an aortic impedance and/or aortic compliance of a patient based on blood pressure data for the patient, and using a first subset of those cardiovascular metrics supplemented by an additive factor, the present solution enables reliable cardiac monitoring for patients in a clinical setting. Moreover, by determining the additive factor based on a meta-parameter including a weighted sum of combinatorial parameters that include a second subset of the cardiovascular metrics, the present solution advantageously further enables the reliable determination of CO and/or SV for patients whether in a normal hemodynamic or hyperdynamic condition.

From the above description it is manifest that various techniques can be used for implementing the concepts described in the present disclosure without departing from the scope of those concepts. Moreover, while the concepts have been described with specific reference to certain implementations, a person of ordinary skill in the art would recognize that changes can be made in form and detail without departing from the scope of those concepts. As such, the described implementations are to be considered in all respects as illustrative and not restrictive. It should also be understood that the present disclosure is not limited to the particular implementations described herein, but many rearrangements, modifications, and substitutions are possible without departing from the scope of the present disclosure.

**Claims**

1.  A cardiac monitoring system (100) for cardiac monitoring of a patient, in particular for determining a stroke volume of the patient, the cardiac monitoring system comprising:

    a display (124);
    a blood pressure sensor configured (120) to sense a blood pressure of an artery of the patient and generate a blood pressure signal;
    an analog-to-digital converter (106) configured to receive the blood pressure signal and convert the blood pressure signal to blood pressure data in digital form; and
    a hardware processor (102) configured to execute a software code (110) to:

        identify a plurality of cardiovascular metrics (112) of the patient based on the blood pressure data, the plurality of cardiovascular metrics (112) including at least one of an aortic impedance and an aortic compliance of the patient; and
        determine a stroke volume of the patient using a first subset of the plurality of cardiovascular metrics (112) and an additive factor ($\Psi$), the first subset of the plurality of cardiovascular metrics (112) including the at least one of the aortic impedance and the aortic compliance of the patient;
        wherein the additive factor ($\Psi$) comprises a meta-parameter including a weighted sum of combinatorial parameters, each combinatorial parameter including a second subset of the plurality of cardiovascular metrics (112); and wherein
        the additive factor ($\Psi$) comprises a numerator which is a product of the meta-parameter and a standard deviation of an arterial blood pressure waveform of the patient, and a denominator including a mean arterial blood pressure of the patient.

2.  The cardiac monitoring system of claim 1, wherein the hardware processor (102) is configured to execute the software code to determine the stroke volume of the patient using a pulse contour method.

3.  The cardiac monitoring system of claim 1, wherein the denominator of the additive factor is expressed as (MAP - I), where MAP is the mean arterial blood pressure and I is an integer less than 10.

4.  The cardiac monitoring system of claim 1, wherein a cardiovascular metric (112) included in at least one of the combinatorial parameters of the meta-parameter is raised to an exponential power greater than 1.

5.  The cardiac monitoring system of claim 1, wherein a cardiovascular metric (112) included in at least one of the combinatorial parameters of the meta-parameter is raised to a negative exponential power.

6. The cardiac monitoring system of claim 1, wherein a cardiovascular metric (112) included in at least one of the combinatorial parameters of the meta-parameter comprises a standard deviation of an arterial blood pressure waveform (246a, 300A, 300B, 546, 746) of the patient.

7. The cardiac monitoring system of claim 1, wherein a cardiovascular metric (112) included in at least one of the combinatorial parameters of the meta-parameter comprises a pulse rate of the patient.

8. The cardiac monitoring system of claim 1, wherein a cardiovascular metric (112) included in at least one of the combinatorial parameters of the meta-parameter comprises a mean arterial blood pressure of the patient.

9. The cardiac monitoring system of claim 1, wherein a cardiovascular metric (112)included in at least one of the combinatorial parameters of the meta-parameter comprises a skewness of an arterial blood pressure of the patient.

10. The cardiac monitoring system of claim 1, wherein a cardiovascular metric (112) included in at least one of the combinatorial parameters of the meta-parameter comprises a kurtosis of an arterial blood pressure of the patient.

11. The cardiac monitoring system of claim 1, wherein a cardiovascular metric (112) included in at least one of the combinatorial parameters of the meta-parameter is directly proportional to a body surface area of the patient.

12. The cardiac monitoring system of claim 1, wherein a cardiovascular metric (112) included in at least one of the combinatorial parameters of the meta-parameter is inversely proportional to a body surface area of the patient.

13. The cardiac monitoring system of claim 1, wherein a cardiovascular metric (112) included in at least one of the combinatorial parameters of the meta-parameter comprises a systolic area of an arterial blood pressure waveform (246a, 300A, 300B, 546, 746) of the patient.

14. The cardiac monitoring system of claim 1, wherein a cardiovascular metric (112) included in at least one of the combinatorial parameters of the meta-parameter comprises a systolic time of an arterial blood pressure waveform (246a, 300A, 300B, 546, 746) of the patient.

15. The cardiac monitoring system of claim 1, wherein a cardiovascular metric (112) included in at least one of the combinatorial parameters of the meta-parameter comprises a diastolic time of an arterial blood pressure waveform (246a, 300A, 300B, 746) of the patient.


**Patentansprüche**

1. Herzüberwachungssystem (100) für die Herzüberwachung eines Patienten, insbesondere zum Bestimmen eines Schlagvolumens des Patienten, wobei das Herzüberwachungssystem umfasst:

   eine Anzeige (124);
   einen Blutdrucksensor (120), der dazu eingerichtet ist, einen Blutdruck einer Arterie des Patienten zu erfassen und ein Blutdrucksignal zu erzeugen;
   einen Analog-Digital-Umsetzer (106), der dazu eingerichtet ist, das Blutdrucksignal zu empfangen und das Blutdrucksignal in Blutdruckdaten in digitaler Form umzuwandeln; und
   einen Hardwareprozessor (102), der dazu eingerichtet ist, einen Softwarecode (110) auszuführen, um:

   eine Vielzahl kardiovaskulärer Kennzahlen (112) des Patienten auf der Grundlage der Blutdruckdaten zu identifizieren, wobei die Vielzahl kardiovaskulärer Kennzahlen (112) mindestens eine von einer Impedanz der Aorta und einer Compliance der Aorta des Patienten umfasst; und
   ein Schlagvolumen des Patienten unter Verwendung einer ersten Untergruppe der Vielzahl kardiovaskulärer Kennzahlen (112) und eines additiven Faktors ($\Psi$) zu bestimmen, wobei die erste Untergruppe der Vielzahl kardiovaskulärer Kennzahlen (112) mindestens eine von der Impedanz der Aorta und der Compliance der Aorta des Patienten umfasst;
   wobei der additive Faktor ($\Psi$) einen Metaparameter umfasst, der eine gewichtete Summe kombinatorischer Parameter umfasst, wobei jeder kombinatorische Parameter eine zweite Untergruppe der Vielzahl kardiovaskulärer Kennzahlen (112) umfasst; und wobei
   der additive Faktor ($\Psi$) einen Zähler, der ein Produkt des Metaparameters und einer Standardabweichung einer Wellenform des arteriellen Blutdrucks des Patienten ist, und einen Nenner umfasst, der einen mittleren arteriellen Blutdruck des Patienten umfasst.

2. Herzüberwachungssystem nach Anspruch 1, wobei der Hardwareprozessor (102) dazu eingerichtet ist, den Softwarecode auszuführen, um das Schlagvolumen des Patienten unter Verwendung eines Pulskonturverfahrens zu bestimmen.

3. Herzüberwachungssystem nach Anspruch 1, wobei der Nenner des additiven Faktors als (MAP - I) ausgedrückt ist, wobei MAP der mittlere arterielle Blutdruck ist und I eine Ganzzahl kleiner als 10 ist.

4. Herzüberwachungssystem nach Anspruch 1, wobei eine kardiovaskuläre Kennzahl (112), die in mindestens einem der kombinatorischen Parameter des Metaparameters enthalten ist, zu einer Potenz größer 1 erhoben ist.

5. Herzüberwachungssystem nach Anspruch 1, wobei eine kardiovaskuläre Kennzahl (112), die in mindestens einem der kombinatorischen Parameter des Metaparameters enthalten ist, zu einer negativen Potenz erhoben ist.

6. Herzüberwachungssystem nach Anspruch 1, wobei eine kardiovaskuläre Kennzahl (112), die in mindestens einem der kombinatorischen Parameter des Metaparameters enthalten ist, eine Standardabweichung einer Wellenform des arteriellen Blutdrucks (246a, 300A, 300B, 546, 746) des Patienten umfasst.

7. Herzüberwachungssystem nach Anspruch 1, wobei eine kardiovaskuläre Kennzahl (112), die in mindestens einem der kombinatorischen Parameter des Metaparameters enthalten ist, eine Herzfrequenz des Patienten umfasst.

8. Herzüberwachungssystem nach Anspruch 1, wobei eine kardiovaskuläre Kennzahl (112), die in mindestens einem der kombinatorischen Parameter des Metaparameters enthalten ist, einen mittleren arteriellen Blutdruck des Patienten umfasst.

9. Herzüberwachungssystem nach Anspruch 1, wobei eine kardiovaskuläre Kennzahl (112), die in mindestens einem der kombinatorischen Parameter des Metaparameters enthalten ist, eine Schiefe eines arteriellen Blutdrucks des Patienten umfasst.

10. Herzüberwachungssystem nach Anspruch 1, wobei eine kardiovaskuläre Kennzahl (112), die in mindestens einem der kombinatorischen Parameter des Metaparameters enthalten ist, eine Wölbung eines arteriellen Blutdrucks des Patienten umfasst.

11. Herzüberwachungssystem nach Anspruch 1, wobei eine kardiovaskuläre Kennzahl (112), die in mindestens einem der kombinatorischen Parameter des Metaparameters enthalten ist, direkt proportional zu einer Körperoberfläche des Patienten ist.

12. Herzüberwachungssystem nach Anspruch 1, wobei eine kardiovaskuläre Kennzahl (112), die in mindestens einem der kombinatorischen Parameter des Metaparameters enthalten ist, umgekehrt proportional zu einer Körperoberfläche des Patienten ist.

13. Herzüberwachungssystem nach Anspruch 1, wobei eine kardiovaskuläre Kennzahl (112), die in mindestens einem der kombinatorischen Parameter des Metaparameters enthalten ist, einen systolischen Bereich einer Wellenform des arteriellen Blutdrucks (246a, 300A, 300B, 546, 746) des Patienten umfasst.

14. Herzüberwachungssystem nach Anspruch 1, wobei eine kardiovaskuläre Kennzahl (112), die in mindestens einem der kombinatorischen Parameter des Metaparameters enthalten ist, eine systolische Zeit einer Wellenform des arteriellen Blutdrucks (246a, 300A, 300B, 546, 746) des Patienten umfasst.

15. Herzüberwachungssystem nach Anspruch 1, wobei eine kardiovaskuläre Kennzahl (112), die in mindestens einem der kombinatorischen Parameter des Metaparameters enthalten ist, eine diastolische Zeit einer Wellenform des arteriellen Blutdrucks (246a, 300A, 300B, 546, 746) des Patienten umfasst.

**Revendications**

1. Système de surveillance cardiaque (100) pour la surveillance cardiaque d'un patient, en particulier pour la détermination d'un volume systolique du patient, le système de surveillance cardiaque comportant :

   un dispositif d'affichage (124) ;
   un capteur de tension artérielle (120) conçu pour détecter une tension artérielle d'une artère du patient et pour générer un signal de tension artérielle ;
   un convertisseur analogique-numérique (106) conçu pour recevoir le signal de tension artérielle et pour convertir le signal de tension artérielle en données de tension artérielle sous forme numérique ; et
   un processeur matériel (102) conçu pour exécuter un code logiciel (110) afin de :

   identifier une pluralité de métriques cardiovasculaires (112) du patient sur la base des données de tension artérielle, la pluralité de métriques cardiovasculaires (112) comprenant au moins un élément parmi une impédance aortique et une compliance aortique du patient ; et
   déterminer un volume systolique du patient à l'aide d'un premier sous-ensemble de la pluralité de métriques cardiovasculaires (112) et d'un facteur additif ($\Psi$), le premier sous-ensemble de la pluralité de métriques cardiovasculaires (112) comprenant ledit au moins un élément parmi l'impédance aortique et la compliance aortique du patient ;
   le facteur additif ($\Psi$) comportant un méta-paramètre comprenant une somme pondérée de paramètres combinatoires, chaque paramètre combinatoire comprenant un second sous-ensemble de la pluralité de métriques cardiovasculaires (112) ; et
   le facteur additif ($\Psi$) comportant un numérateur qui est un produit du méta-paramètre et d'un écart-type d'une forme d'onde de tension artérielle du patient et un dénominateur comprenant une tension artérielle moyenne du patient.

2. Système de surveillance cardiaque selon la revendication 1, le processeur matériel (102) étant conçu pour exécuter le code logiciel afin de déterminer le volume systolique du patient à l'aide d'un procédé de contour de l'onde de pouls.

3. Système de surveillance cardiaque selon la revendication 1, le dénominateur du facteur additif étant exprimé en tant que (MAP-I), MAP étant la tension artérielle moyenne et I étant un nombre entier inférieur à 10.

4. Système de surveillance cardiaque selon la revendication 1, une métrique cardiovasculaire (112) comprise dans au moins l'un des paramètres combinatoires du méta-paramètre étant élevée à une puissance exponentielle supérieure à 1.

5. Système de surveillance cardiaque selon la revendication 1, une métrique cardiovasculaire (112) comprise dans au moins l'un des paramètres combinatoires du méta-paramètre étant élevée à une puissance exponentielle négative.

6. Système de surveillance cardiaque selon la revendication 1, une métrique cardiovasculaire (112) comprise dans au moins l'un des paramètres combinatoires du méta-paramètre comportant un écart-type d'une forme d'onde de tension artérielle (246a, 300A, 300B, 546, 746) du patient.

7. Système de surveillance cardiaque selon la revendication 1, une métrique cardiovasculaire (112) comprise dans au moins l'un des paramètres combinatoires du méta-paramètre comportant une fréquence du pouls du patient.

8. Système de surveillance cardiaque selon la revendication 1, une métrique cardiovasculaire (112) comprise dans au moins l'un des paramètres combinatoires du méta-paramètre comportant une tension artérielle moyenne du patient.

9. Système de surveillance cardiaque selon la revendication 1, une métrique cardiovasculaire (112) comprise dans au moins l'un des paramètres combinatoires du méta-paramètre comportant une asymétrie d'une tension artérielle du patient.

10. Système de surveillance cardiaque selon la revendication 1, une métrique cardiovasculaire (112) comprise dans au moins l'un des paramètres combinatoires du méta-paramètre comportant un kurtosis d'une tension artérielle du patient.

**11.** Système de surveillance cardiaque selon la revendication 1, une métrique cardiovasculaire (112) comprise dans au moins l'un des paramètres combinatoires du méta-paramètre étant directement proportionnelle à une aire de surface corporelle du patient.

**12.** Système de surveillance cardiaque selon la revendication 1, une métrique cardiovasculaire (112) comprise dans au moins l'un des paramètres combinatoires du méta-paramètre étant inversement proportionnelle à une aire de surface corporelle du patient.

**13.** Système de surveillance cardiaque selon la revendication 1, une métrique cardiovasculaire (112) comprise dans au moins l'un des paramètres combinatoires du méta-paramètre comportant une aire systolique d'une forme d'onde de tension artérielle (246a, 300A, 300B, 546, 746) du patient.

**14.** Système de surveillance cardiaque selon la revendication 1, une métrique cardiovasculaire (112) comprise dans au moins l'un des paramètres combinatoires du méta-paramètre comportant une durée systolique d'une forme d'onde de tension artérielle (246a, 300A, 300B, 546, 746) du patient.

**15.** Système de surveillance cardiaque selon la revendication 1, une métrique cardiovasculaire (112) comprise dans au moins l'un des paramètres combinatoires du méta-paramètre comportant une durée diastolique d'une forme d'onde de tension artérielle (246a, 300A, 300B, 546, 746) du patient.

# Fig. 1

**Cardiac Monitoring System**

Hardware Processor — 102

Memory 104

Software Code 110

Metrics — 112

106

ADC

144

DAC — 108

User Interface — 128 — 126

Display 124

122

130

120

142 — 140

# Fig. 2A

# Fig. 2B

# Fig. 3A

300A

Sys_Area

# Fig. 3B

300B

Sys_T

# Fig. 4A

# Fig. 4B

# Fig. 5

# Fig. 6

650

Convert a blood pressure signal corresponding to a blood pressure of an artery of a patient to a blood pressure data in digital form
～652

Identify cardiovascular metrics of the patient, including one or more of an aortic impedance and aortic compliance of the patient, based on the blood pressure data
～654

Determine a stroke volume (SV) of the patient using a first subset of the cardiovascular metrics and an additive factor, the additive factor being based on a meta-parameter including a weighted sum of combinatorial parameters, each combinatorial parameter including a second subset of the cardiovascular metrics
～656

# Fig. 7

700

712

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20140163401 A1
- EP 2416701 A2
- US 89687313
- US 896873